# EUROPEAN PATENT APPLICATION

(11) **EP 2 322 926 A1**
(43) Date of publication of application: **18.05.2011**
(21) Application number: 09014100.3
(22) Date of filing: 11.11.2009
(51) Int. Cl.: G01N 33/50, G01N 33/564

(54) **Stabilized open form transglutaminase as a diagnostic indicator for autoimmune diseases**

(71) Applicant: Zedira GmbH, 64293 Darmstadt (DE)
(72) Inventor: Hils, Martin Dr., 64295 Darmstadt (DE); Pasternack, Ralf Dr., 64347 Griesheim (DE); Weber, Johannes, 64295 Darmstadt (DE)
(74) Representative: Arth, Hans-Lothar

(57) **Abstract**

The present invention relates to the diagnosis of disorders or dysfunctions characterized by autoimmune responses to the enzyme class of transglutaminases. The present invention provides a novel open structure of the transglutaminases in a stabilized form which renders new epitopes accessible for antibody-binding.

SEQUENCE LISTING

<110>  Zedira GmbH

<120>  Stabilized open form transglutaminase as a diagnostic indicator
       for autoimmune diseases

<130>  ZED-P03073EP

<140>  EP1111111
<141>  2009-11-11

<160>  1

<170>  PatentIn version 3.3

<210>  1
<211>  33
<212>  PRT
<213>  Triticum aestivum

<400>  1

Leu Gln Leu Gln Pro Phe Pro Gln Pro Gln Leu Pro Tyr Pro Gln Pro
1               5                   10                  15

Gln Leu Pro Tyr Pro Gln Pro Gln Leu Pro Tyr Pro Gln Pro Gln Pro
            20                  25                  30

Phe

## Description

The present invention relates to the diagnosis of disorders or dysfunctions characterized by autoimmune responses to the enzyme class of transglutaminases. The present invention provides a novel open structure of the transglutaminases in a stabilized form which renders new epitopes accessible for antibody-binding.

### Background of the invention

Transglutaminases (TG) are enzymes that exist in higher vertebrates including the human body as a family of nine members, including the inactive structural protein band 4.2. They fulfil a large variety of physiological functions e.g. in coagulation, inflammation, cell differentiation, apoptosis, cell-cell and cell-matrix interactions, and wound healing. In the presence of Ca²⁺ transglutaminases may develop their catalytic activity, the formation of inter- and intra-molecular γ-glutamyl-ε-lysine bonds, also called iso-peptide bonds, between the side chains of peptidic glutamine and lysine. In general the results of this activity are high-molecular weight protein aggregates. Transglutaminases 1, 3 and 5 are mainly involved in skin-formation, factor XIII (plasma-transglutaminase) is active in blood coagulation and wound healing, transglutaminase 6 has mainly been found in neuronal tissues and transglutaminase 4 in semen fluid. Transglutaminase 2 (tissue transglutaminase, TG2) is ubiquitously present in various tissues. Besides its cross-linking activity TG2 may also deamidate the carboxyamide group of peptidic glutamine, especially under slightly acidic conditions as well as in the absence of lysine or other primary amines. As the deamidation product is a carboxyl group, a negative charge is introduced into the peptide which results in a significant change of peptide properties. Further TG2 has GTP-hydrolysing activity and functions as a G-Protein. Even kinase activity is described. TG2 plays a role in several diseases like celiac disease, fibrosis, cancer, and neurological disorders.

In celiac disease, a chronic inflammation of the small intestine and an autoimmune disease, TG2 plays a multifunctional role. Digestion of proteins present in our food is performed by proteolytical cleavage to amino acids and small peptides, which can be resorbed by the intestinal mucosa. Cereal proteins like gluten show a high content of the amino acid proline. But the enzymes responsible for protein degradation in the digestive tract are not able to hydrolyse proline-rich proteins into amino acids and small peptides. Consequently rather long peptides are present in the mucosa. Due to their size they cannot be resorbed, but they are still able to pass the epithelium and to reach the so called lamina propria, a connecting tissue between epithelium and muscular tissue. In the lamina propria extracellular TG2 is present. Now an essential step in celiac disease pathophysiology takes place: TG2 deamidates gliadin peptides. In their deamidated form gliadin peptides are recognized by the HLA-DQ2 and HLA-DQ8 receptors of immune cells. This recognition leads to the triggering of the inflammatory response which in the final stage of the disease manifests in a complete villous atrophy of the intestinal mucosa. TG2 not only catalyses gliadin deamidation, it serves also as autoantigen in celiac disease. Patients who suffer from celiac disease develop autoantibodies against TG2. If patients successfully follow a strict gluten free diet anti-TG2-autoantibody titers decrease. Therefore TG2 is not only used as antigen for autoantibody-detection in the diagnosis of celiac disease, but also in the follow up and control of the gluten free diet. TG2-autoantibodies are also found in other diseases like diabetes type 1 or psoriasis and may therefore be used in non-celiac disease diagnostics too.

Interestingly in other indications developing along or in consequence to celiac disease further autoantibodies against transglutaminases can be found: Dermatitis herpetiformis, a gluten sensitive skin disorder is characterized by the presence of autoantibodies against epidermal transglutaminase (TG3) whereas in glutensensitive ataxia autoantibodies against neuronal transglutaminase (TG6) have been found.

Objective of the present invention is to provide a more sensitive method for detecting autoimmune diseases characterized by the presence of transglutaminase specific autoantibodies.

The objective of the present invention is solved by the teaching of the independent claims. Further advantageous features, aspects and details of the invention are evident from the dependent claims, the description, the figures and the examples of the present application.

### Description of the invention

The present invention surprisingly found, that the use of the open form transglutaminase for detection of autoantibodies leads to higher titers compared to the closed form of the transglutaminase and reveals positive titers in patients with titers against the closed TG form below the cut-off. This invention is of immense impact on diagnosis, antibody isolation and immune cell characterization.

The transglutaminases change their tertiary structure upon substrate binding. Inactive non-substrate binding TG shows a closed form which upon substrate binding changes to an open form. Both forms the closed form without substrate and the open form with substrate are recognized by autoantibodies. It is assumed that autoantibodies for the closed form as well as for the open form exist.

However it was most surprisingly found that the open form of the TG can be used to detect autoantibodies in much higher sensitivity in comparison to the closed form. Consequently, much more sensitive and precise methods for detecting autoantibodies against the open form of the TG were developed and are subject of the present invention in order to much more precisely detect and diagnose diseases associated with the presence of autoantibodies for the open form transglutaminase.

Thus the present invention relates to a method for the diagnosis of an autoimmune disorder or an intestinal disorder or a skin disorder or a neurological disorder or other diseases, wherein the open form transglutaminase or antigenically active fragments of the open form transglutaminase are used for the detection of the presence of autoantibodies specific for the open form transglutaminase or antigenically active fragments of the open form transglutaminase.

The open form of the transglutaminase or a fragment of the open form of a transglutaminase which has comparable activity to the open form transglutaminase, thus an antigenically active fragment of this open form transglutaminase is generated by reacting the transglutaminase or the antigenically active fragment of the transglutaminase with an inhibitor comprising a backbone, a peptide backbone or a peptidomimetic backbone and a thiol reactive group able to form a covalent bond to the thiol group of the cysteine in the active site of the TG. The inhibitor might also react with other cysteines which are not located in the active side of the TG but the inhibitor must be able to react with the cysteine in the active site of the respective TG. In order to make the inhibitor more regioselective, the thiol reactive group of the inhibitor is preferably attached to a backbone, a peptide backbone or peptidomimetic backbone or any synthetic backbone which has affinity to the active site in order to increase regioselectivity of the inhibitor molecule. In order to generate the open form of a TG it is important that the inhibitor attaches covalently to the thiol group of the cysteine within the active site of the TG. Consequently, inhibitors are preferred which have a backbone attached to a thiol reactive group which has certain similarity to the natural substrates of the TG. Such backbones are preferably peptide-like backbones, i.d. backbones which comprise amide bonds and at least one carbon chain attached to the carbonyl group of the amide bond or at least one optionally substituted carbon atom between two amide bonds.

The autoimmune disorder can be selected from the group comprising or consisting of Addisons's disease, alopecia areata, ankylosing spondylitis, antiphosholipid antibody syndrome, autoimmune hemolytic anemia, autoimmune hepatitis, autoimmune inner ear disease, autoimmune lymphoproliferative syndrome, autoimmune thrombocytopenic purpura, immune thrombocytopenic purpura, balo disease, Behcet's disease, bullous pemphigoid, cardiomyopathy, chronic fatigue immune dysfunction syndrome, chronic inflammatory demyelinating polyneuropathy, Cicatricial pemphigoid, cold agglutinin disease, CREST syndrome, Crohn's disease, Degos disease, dermatomyositis, dermatomyositis-juvenile, discoid lupus, essential mixed cryoglobulinemia, fibromyalgia, Goodpasture's syndrome, Graves disease, Guillain-Bar6, Hashimoto thyroiditis, idiopathic pulmonary fibrosis, idiopathic thrombocytopenia purpura, IgA nephropathy, insulin-dependent diabetes, juvneile arthritis, lichen planus, lupus, Ménière disease, mixed connective tissue disease, multiple sclerosis, myasthenia gravis, PANDAS, pemphigus vulgaris, pernicious anemia, polyarteritis nodosa, polychondritis, polyglandular syndromes, polymyalgia rheumatica, polymyositis, dermatomyositis, primary agammaglobulinemia, primary biliary cirrhosis, psoriasis, Raynaud phenomenon, Reiter's syndrome, rheumatic fever, rheumatoid arthritis, sarcoidosis, scleroderma, sclerosing cholangitis, Sjögren's syndrome, Takayasu's arteritis, temporal arteritis, giant cell arteritis, ulcerative colitis, uveitis vasuclitis, vitiligo, Wegener's granulomatosis, neurological disorder, paraneoplastic neurological syndrome, cereal protein sensitivity disorder, gluten sensitive disorders, celiac disease, dermatitis herpetiformis, Morbus Duhring, diabetes mellitus type 1, anxiety, depression, brainstem encephalitis, cerebral vasculitis, chorea, dementia, epilepsy, cerebral calcifications, headache with white matter abnormalities, neuromyotonia, myasthenia gravis, myopathy, peripheral neuropathy, progressive multifocal leukoencephalopathy, progressive myoclonic encephalopathy, schizophreniform disorder, stiff-person syndrome, ataxia, ataxia with myoclonus, encephalitis, polymyositis, epilepsy with occipital cerebral calcifications, peripheral neuropathy, loss of sensation or muscle weakness, paraneoplastic cerebellar degeneration, paraneoplastic encephalomyelitis, paraneoplastic opsoclonus-myoclonus, cancer associated retinopathy, paraneoplastic stiff-man syndrome, paraneoplastic necrotizing myelopathy, a motor neuron syndrome, amyotrophic lateral sclerosis, subacute motor neuronopathy, subacute sensory neuronopathy, autonomic neuropathy, acute sensorimotor neuropathy, polyradiculoneuropathy, brachial neuritis, chronic sensorimotor neuropathy, a sensorimotor neuropathy associated with plasma cell dyscrasias, vasculitic neuropathy and neuromyotonia.

The present invention is most useful for the diagnosis of an autoimmune disorder or autoimmune disease and especially of gluten sensitive disorders, celiac disease, dermatitis herpetiformis and gluten sensitive ataxia.

In other words the present invention relates to a method for the diagnosis of an autoimmune disorder, wherein at least one open form transglutaminase or at least one antigenically active fragment of the open form transglutaminase is used for the detection of the presence of autoantibodies specific for the open form transglutaminase or specific for the antigenically active fragment of the open form transglutaminase.

The transglutaminases or open form transglutaminases used within the inventive method are selected from the group comprising or consisting of TG1, TG2, TG3, TG4, TG5, TG6, TG7 or coagulation factor XIII.

The antigenically active fragments of the transglutaminases or of the open form transglutaminases are selected from the group comprising or consisting of antigenically active fragments of TG1, TG2, TG3, TG4, TG5, TG6, TG7 or coagulation factor XIII.

The term "antigenically active fragment" as used herein refers to a protein fragment that is able to elicit an antibody response and is able to be bound by an antibody. In regard to a TG, an "antigenically active fragment" can be further defined as an arrangement of amino acid residues forming part of the surface of the TG, in particular the surfaces exposed upon enzyme activation, either in the form of a continuous peptide sequence or amino acids held together by an artificial backbone that mimics their arrangement in the native protein. An "antigenically active fragment" can also be a portion of a TG with proteins or derived peptides bound to its active site, which constitute common T cell epitopes. Examples for such proteins are proteins of the extracellular matrix like collagen, fibronectin, vitronectin, osteonectin or villamentin or plasma proteins like α2-antiplasmin, fibrin and fibrinogen or food-proteins like gliadin. An example for a gliadin derived peptide is the alpha-gliadin derived 33-mer LQLQPFPQPQLPYPQPQLPYPQPQLPYPQPQPF [accession number AJ133612 (amino acids 57-89)].

The methods of the present invention can also be performed using more than one open form transglutaminase and/or more than one antigenically active fragment of the open form transglutaminase at the same time within the method. Thus, mixtures of transglutaminases (TGs) can be used within the inventive methods. Furthermore the detection of, for example, transglutaminase 2 autoantibodies can be combined with the detection of autoantibodies to one or more further transglutaminase isoforms such as TG1, TG3, TG4, TG5, TG6, TG7 or coagulation factor XIII.

In the following the invention is described in detail in regard to transglutaminase 2 (TG2) while the invention can be performed with all other TGs in a similar manner. Thus, the present invention is not limited to the use of TG2. TG2 is only used as representative example of all TGs for disclosing the present invention in detail.

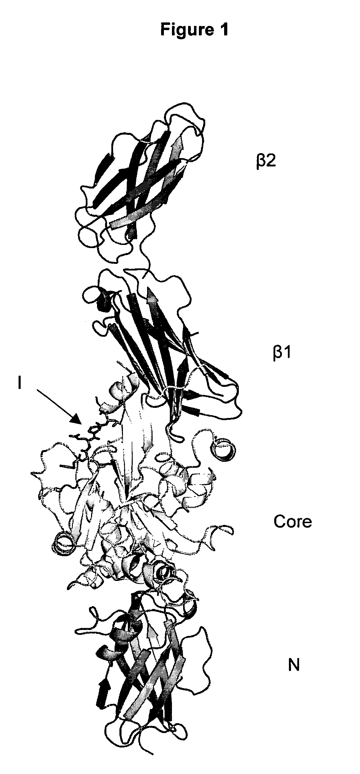

The transglutaminase 2 changes its tertiary structure upon substrate binding. Whereas inactive non-substrate bound TG2 shows a closed more ball-shaped form, the two β-domains turn upon substrate binding resulting in a more longitudinal form as shown in figure 1. This shift in tertiary structure is a common feature of all vertebrate transglutaminases. Thus novel epitopes, which have been covered in the closed form, are now accessible.

For instance, the tissue transglutaminase (TG2) is the autoantigen in celiac disease. The disease is characterized by an elevated titer of autoantibodies against TG2 (IgA and IgG). Although suffering from celiac disease, some patients show very low or no antibody titers at all. In a group of patients the lacking IgA-titer can be explained by a general IgA-deficiency. But there remain celiac disease patients with a biopsy proven celiac disease (intestinal villous atrophy) and without IgA-deficiency, but a TG2-autoantibody negative or doubtful serology.

Thus, the diseases described herein can be detected more precisely and accurate by the use of the open form TG for the detection of autoantibodies against or specifically against said open TG form.

The open form of the transglutaminase is present at the time of binding a substrate to the TG. The TG is in a closed form when no substrate is bound. In order to obtain the TG in its open form a substrate or an inhibitor is added to the TG. Since it is advantageous to obtain a TG stabilized in its open form, inhibitors and preferably irreversible inhibitors are used which react with the TG. Accordingly, in order to obtain the antigenically active fragment of the open form transglutaminase, the antigenically active fragment of the open form transglutaminase is reacted with an inhibitor and preferably an irreversible inhibitor in order to stabilize the open form of the antigenically active fragment of the open form transglutaminase.

As used herein, the term "irreversible inhibitor" refers to an inhibitor which covalently binds to the TG or which binds sufficiently tightly so that no equilibrium will exist. Irreversible inhibitors are most often inhibitors with at least one functional group which is able to form a covalent bond to the target such as a TG so that one inhibitor is required to inactivate one TG enzyme and consequently stabilize the open form of the TG.

Inhibitors based on proteins, e.g. proteins of the extracellular matrix, plasma-proteins or food-proteins, and dipeptidic or larger portions thereof as well as inhibitors comprising a peptide-like backbone, peptidomimetic backbone or any other backbone which has affinity to the active site of the TG or which has similarity with the backbone of the natural substrate in regard to length, number of atoms, tertiary structure, molecular weight, functional groups and/or polarity can be used for the production of the open form of transglutaminase 1, transglutaminase 2, transglutaminase 3, transglutaminase 4, transglutaminase 5, transglutaminase 6, transglutaminase 7 or the coagulation factor XIII.

Suitable inhibitors which are able to form covalent bonds to the TGs are inhibitors with thiol-reactive groups. Thiol-reactive groups are preferably such groups which are electrophil and which are able to undergo a nucleophilic addition reaction with the thiol group (-SH group) of cysteine such as acetylenes, electrophilic olefins, unsaturated aldimines, Michael acceptors, iodoacetamide, N-ethylmaleimide, parachloromercuribenzoic acid, alkyl isocyanates, 3,5-substituted-4,5-dihydroisoxazoles, oxiranes as described in U.S. Pat. No. 5,188,830, moreover imidazoles, pyrazoles, triazoles and tetrazoles as disclosed in U.S. Pat. No. 4,968,713, U.S. Pat. No. 5,019,572, U.S. Pat. No. 5,021,440, U.S. Pat. No. 5,030,644, U.S. Pat. No. 5,047,416, U.S. Pat. No. 5,077,285, U.S. Pat. No. 5,084,444, U.S. Pat. No. 5,098,707, U.S. Pat. No. 5,152,988 and U.S. Pat. No. 5,177,092, as well as the compound of the general formula 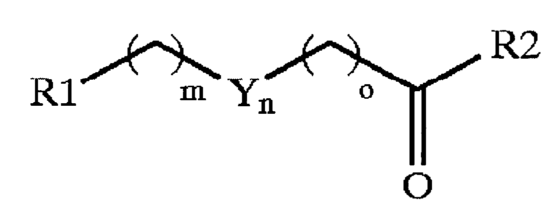 as disclosed in US 2002132776 A1 and other similar compounds with comparable reactivity in regard to thiol groups.

Preferred inhibitors which form covalent bonds to the TGs are inhibitors which comprise a peptide backbone or peptidomimetic backbone and a Michael acceptor moiety as reactive group. This Michael acceptor moiety is preferably a side chain bound to the peptidic or peptidomimetic backbone. Michael acceptor systems normally consist of a carbon-carbon double bond in conjugation to a carbonyl group.

Preferred inhibitors are represented by the general formula [TGI1]:

acceptor-substituted double bond-(CO)ₘ-C₂H₄-backbone [TGl1]

wherein m stands for 0 or 1 and preferably m is 0 and
the acceptor-substituted double bond carries at least one electron-drawing residue capable to conjugate with an electronegativity ≥ 2.20 and
the backbone is preferably a peptide or peptidomimetic from at least two amino acids and preferably a tetrapeptide or at least a dipeptidomimetic and/or the backbone shows at least one amide bond. The acceptor-substituted double bond is preferably a Michael system.

Further preferred inhibitors are represented by the general formula (I), (II) or (III): 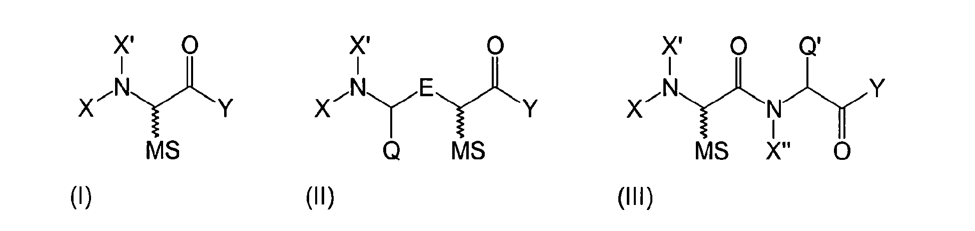 wherein
MS is the acceptor-substituted olefin of the following structure: 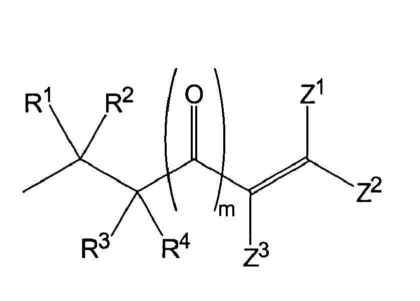 E represents the following group -CH₂-, -CF₂-, -C₂H₄-, -CH₂-CF₂-, -CF₂-CH₂-, -CH=CH-, -CH(OH)-CH₂-, -C(=O)-CH₂-, -CH₂-NH-, -CH₂-O-, -CH(OH)-CH₂-NH-, -P(=O)(OH)-NH-, -P(=O)(OH)-O-, -P(=O)(OH)-S-, -P(=O)(OH)-CH₂-, -CH(OH)-CH₂-NH-, -C(=O)-NH-, -C(=O)-O- or -C(=O)-NX"-;
m is 0 or 1;
the residues **Z¹, Z²,** Z³ independently of each other represent the following groups:
-CO-(C₁-C₆ alkyl), -CO-R⁶, -CO-R⁷, -CO-(C₁-C₆ halogenalkyl), -CO-(C₃-C₁₀ heteroaryl), -CO-(C₆-C₁₅ aryl), -COO-(C₁-C₆ halogenalkyl), -COO-(C₃-C₁₀ heteroaryl), -COO-(C₆-C₁₅ aryl), -COO-(C₁-C₆ alkyl), -COO-R⁸, -COO-R⁹, -CN, -F, -CI, -COOH, -CO-NH(C₁-C₆ alkyl), -CO-N(C₁-C₆ alkyl)(C₁-C₆ alkyl), -CO-NR¹⁰R¹¹, -CO-NH₂, -CO-N(CR¹²R¹³R¹⁴)(CR¹⁵R¹⁶R¹⁷), -CH₂CN, -CH₂F, -CHF₂, -CF₃, -OCF₃, -CH₂-CF₃, -CF₂-CF₃, -NO₂, **-CS-(C₁-C₆** alkyl), -CS-R¹⁸, -CS-R¹⁹, -CS-O-(C₁-C₆ alkyl), -CS-O-R²⁰, -CS-O-R²¹, -CS-N(C₁-C₆ alkyl)(C₁-C₆ alkyl), -CS-NR²²R²³, -CS-NH₂, -CS-N(CR²⁴R²⁵R²6)(CR²⁷R²⁸R²⁹), -SO-R³⁰, -SO-R³¹, -SO₂-R³², -SO₂-R³³, -SO-CR³⁴R³⁵R³⁶, -SO-CR³⁷R³⁸R³⁹, -SO₂-CR⁴⁰R⁴¹R⁴², -SO₂-CR⁴³R⁴⁴R⁴⁵, -SO-N(C₁-C₆ alkyl)(C₁-C₆ alkyl), -SO-NR⁴⁶R⁴⁷, -SO-NH₂, -SO-N (CR⁴⁸R⁴⁹R⁵⁰)(CR⁵¹R⁵²R⁵³), -SO₂-N(C₁-C₆ alkyl)(C₁-C₆ alkyl), -SO₂-NR⁵⁴R⁵⁵, -SO₂-NH₂, -SO₂-N(CR⁵⁶R⁵⁷R⁵⁸)(CR⁵⁹R⁶⁰R⁶¹), -SO₂-OH, -SO₂-OR⁶², -SO₂-CR⁶³R⁶⁴R⁶⁵, -SO₂-OCR⁶⁶R⁶⁷R⁶⁸, -O-P(O)(OH)₂, -O-P(O)(OR⁶⁹)(OR⁷⁰), -O-P(O)(O-C₁-C₆ alkyl)(O-C₁-C₆ alkyl), -P(O)(OR⁷¹)(OR⁷²), -P(O)(O-C₁-C₆ alkyl)(O-C₁-C₆ alkyl), -CF₂-P(O)(OR⁷³)(OR⁷⁴), -CF₂-P(O)(O-C₁-C₆ alkyl)(O-C₁-C₆ alkyl), wherein at least one of the residues Z¹, Z², Z³ is different from hydrogen;
the residues **Z¹** and **Z²** together may also represent a residue -CO-O-CO-CH₂-, - CO-O-CH₂-CH₂-,
the residues **Z²** and **Z³** together may also represent a residue -CO-Z'-CH₂-, -COO-CH₂-, -CO-CH₂-CH₂, -CO-O-CO-, -CO-NH-CO- or -Z'-CH₂-CH₂- , wherein
Z' represents one of the following groups: -CH₂-, -CF₂-, -C₂H₄-, -CF₂-CH₂-, -CH₂-CH₂-, -O-, -O-CH₂-, -NH- or -NH-CH₂-;
**Q** and **Q'** independently of each other represent a side chain residue of a natural amino acid; or **Q** together with **X'** forms a propylenyl residue; or **Q'** together with **X"** forms a propylenyl residue;
**Y** represents a hydroxy group, amino group, C₁-C₆ alkylamino group, C₁-C₆ dialkylamino group, C₁-C₆ alkoxy group, C₆-C₁₉ aryloxy group, C₁-C₆ alkyl group, C₁-C₆ halogenalkyl group, C₃-C₁₀ heteroaryl group or a C₆-C₁₅ aryl group; or Y represents a peptide residue of up to 6 amino acids and bound via an amide bond, the C-terminal carbonyl function of which peptide residue carries a hydroxy group, amino group, C₁-C₆ alkylamino group, C₁-C₆ dialkylamino group, C₁-C₆ alkoxy group, C₁-C₆ alkyl group, C₁-C₆ halogenalkyl group, C₃-C₁₀ heteroaryl group or a C₆-C₁₅ aryl group; or Y represents a peptidomimetic residue of up to 60 carbon atoms and
**X"** represents hydrogen or a C₁-C₆ alkyl group; and
**-NXX'** is an amino group, C₁-C₁₀ alkylamino group, C₆-C₁₂ aralkyloxycarbonyl amino group, C₁-C₁₀ dialkylamino group, C₂-C₆ nitrogen heterocycle or a C₃-C₅ nitrogen heteroaryl group; or the group -NXX' is part of a peptidomimetic residue of up to 60 carbon atoms
or
**X'** represents hydrogen or a C₁-C₆ alkyl group; and
**X** represents a peptide residue of up to 6 amino acids and bound via an amide bond, the N-terminus of which peptide residue carries an amino group, C₁-C₁₀ alkylamino group, C₁-C₈-alkyloxycarbonylamino group C₆-C₁₂ aralkyloxycarbonyl amino group, C₁-C₁₀ dialkylamino group, C₂-C₆ nitrogen heterocycle or a C₃-C₅ nitrogen heteroaryl group;
wherein any of the C₁-C₆ alkoxy groups, C₁-C₆ alkyl groups, C₁-C₁₀ alkylamino groups, C₁-C₈-alkyloxycarbonylamino groups, C₆-C₁₂ aralkyloxycarbonyl amino group, C₁-C₁₀ dialkylamino groups, C₂-C₆ nitrogen heterocycles as well as C₃-C₅-nitrogen heteroaryl groups can be independently substituted with up to 5 residues selected from R⁸⁰, R⁸¹, R⁸², R⁸³, R⁸⁴,
wherein the residues **R¹ - R⁸⁴** have the meanings as disclosed in WO 2008055488 A1 and stereoisomeric forms, E/Z isomers, enantiomers, enantiomeric mixtures, diastereomers, diastereomeric mixtures, racemates, tautomers, anomers, keto-enol forms, betaine forms, prodrugs, solvates, hydrates as well as pharmacologically acceptable salts of the aforementioned compounds.

The above-mentioned Michael acceptor inhibitors, their synthesis and use are explicitly described in WO 2008055488 A1. It is especially referred to pages 3 to 38 of WO 2008055488 A1.

Furthermore, the present invention describes the production of TG2 in a stabilized open form preferably by the use of the irreversible inhibitors disclosed above. TG2 was produced by recombinant DNA-technology in *Escherichia coli* and purified to homogeneity. Subsequently it was brought to reaction with Z-PIMen(OEt)-QPL-OMe which is the compound N'-benzyloxycarbonyl-{[(E)-(L)-6-amino-hept-2-ene-dicarboxylic acid]-1-ethanoyl}-L-glutaminyl-L-prolinyl-L-leucine methyl ester (compound 2) synthesized in our labs according to the procedure described in WO 2008055488 A1 on page 68). By a further purification step excess Z-PIMen(OEt)-QPL-OMe was separated. The resulting TG2 - Z-PIMen(OEt)-QPL-OMe-adduct was crystallized. X-ray-structure analysis proved that TG2 is present in its open form after reaction with Z-PIMen(OEt)-QPL-OMe.

Surprisingly it was found that TGs or antigenically active fragments of TGs stabilized by inhibitors and preferably irreversible inhibitors such as the inhibitors disclosed above are present in their open form which is much more sensitive in regard to the detection of autoantibodies against TGs or antigenically active fragments of TGs than the TGs in their closed form or the antigenically active fragments of the TGs in their closed form.

Another aspect of the present invention relates to the use of a gluten-derived peptide with a thiol reactive group or preferably with a Michael acceptor residue as an inhibitor for the production of the open form transglutaminase. Such an adduct of a TG and a gluten-derived peptide is again preferably prepared by reacting the TG with a reactive group such as a Michael acceptor moiety of the gluten-derived peptide. Such an adduct of a TG such as TG2 with a gluten-derived peptide is useful for the simultaneous detection of anti-gliadin antibodies and anti open form transglutaminase autoantibodies.

Consequently the present invention also relates to the use of a gluten-derived peptide with a thiol reactive residue or a Michael acceptor residue as an inhibitor for the production of the open form of a TG such as TG2, wherein the open form TG is useful for the simultaneous detection of anti-gliadin antibodies and anti open form TG2 autoantibodies.

A further aspect of the present invention is directed to immobilized open form TGs which are immobilized on, for instance, titer plates, microtiter plates or well plates. In addition the present invention relates to titer plates, microtiter plates or well plates which immobilized open form TGs and/or immobilized antigenically active fragments of open form TGs on their surface and especially within the wells where the detection reaction is performed.

We found that the stabilized open form TG2 can be used for coating of microtiter plates. Sera from blood donors and from celiac disease patients have been subsequently analysed for autoantibodies against the open form TG2 in comparison to commonly used closed form TG2. Surprisingly the sera revealed higher titers for open form TG2 which resulted in a more significant value. In addition some sera could be proofed positive for autoantibodies to open form TG2 which have been diagnosed negative using the closed form TG2 as antigen.

A further aspect of the present invention is directed to a kit for the diagnosis of an autoimmune disorder characterized by the presence of autoantibodies reacting with at least one open form transglutaminase. Such a kit for diagnosis of an autoimmune disorder comprises at least one open form transglutaminase or at least one antigenically active fragment of an open form transglutaminase for the detection of the presence of autoantibodies specific for the open form transglutaminase or the antigenically active fragment of the open form transglutaminase.

These kits can present the transglutaminase or antigenically active fragments thereof in a form suitable for use in, for example, the following methods: EIA/ELISA, LiA, FiA, RIA, IRMA, IEMA/EIA, ILMA, IFMA, immunodiffusion, Western-blot, Dot-blot, immunohistochemistry, protein chips or protein arrays.

Such kits may also comprise titer plates, microtiter plates or well plates with open form transglutaminase or antigenically active fragments of the open form transglutaminase immobilized on the surface of the plate.

One smart way to manufacture titer plates, microtiter plates or well plates with open form transglutaminase or antigenically active fragments of the open form transglutaminase immobilized on the surface of the plate is to use the interaction of biotin and biotin binding compounds. Therefore biotinlyation of the open form transglutaminase or the antigenically active fragments of the open form transglutaminase is achieved by enzymatic or chemical methods and the biotinylated products are bound to surfaces coated with biotin binding compounds. Another possibility is to bind a biotinylated inhibitor to the TG or antigenically active TG fragment in order to obtain open form TG containing the covalently bound biotinylated inhibitor. Said adduct can be immobilized on the surface of titer plates, microtiter plates or well plates by interacting with biotin binding compounds. Consequently another aspect of the present invention is related to the use of a biotinylated inhibitor for the production of open form transglutaminase for site specific immobilization to surfaces coated with biotin binding compounds.

The above-described kits may further comprise a biotinylated open form transglutaminase or a biotinylated antigenically active open form TG fragment for immobilizing the biotinylated open form transglutaminase or biotinylated antigenically active open form TG fragment to a surface coated with biotin binding compounds. Alternatively the above-described kits may further comprise an open form transglutaminase or an antigenically active fragment thereof reacted with a biotinylated inhibitor for immobilizing the open form transglutaminase biotinylated inhibitor product or the antigenically active fragment biotinylated inhibitor product to a surface coated with biotin binding compounds.

Another aspect of the present invention is directed to the production of open form transglutaminase using an inhibitor linked to one or more antigens specific for autoantibodies found in patients suffering from autoimmune diseases not characterized by the presence of autoantibodies against the respective TG. An example would be linking the autoantigen in rheumatoid arthritis (cyclic citrullinated peptide) via the inhibitor to the TG2

The most important aspect of the present invention is that the present invention provides novel antigens for autoimmune diagnostics which yield higher and more significant titers resulting in a better diagnostic sensitivity. Furthermore, the present invention shows that the conformation has an impact on triggering the autoimmune disease and accordingly to detect early stage disease states.

Moreover the invention implicates that not tissue transglutaminase but the covalent adduct between transglutaminase and (irreversibly) bound substrate or inhibitor displays the disease state and correlates to inflammation and villous atrophy.

The open form transglutaminase is used for the isolation of specific antibodies from patient-derived samples, including blood purification (apheresis).

Thus, the present invention also relates to a method for purifying blood, wherein the open form transglutaminase or antigenically active fragments of the open form transglutaminase are used for binding and removing autoantibodies specific for the open form transglutaminase or antigenically active fragments of the open form transglutaminase from the blood. Such a method uses the common dialysis process. The adsorber material used in the extracorporeal adsorber column contains immobilized open form transglutaminase or antigenically active fragments of the open form transglutaminase in order to bind the autoantibodies against the TG or the antigenically active fragment of the TG or the open form transglutaminase or against the antigenically active fragment of the open form transglutaminase thus removing these autoantibodies from the blood.

Consequently open-form specific antibodies are used for therapeutic approaches, where active and therefore open form transglutaminase shall be neutralized, whereas inactive and therefore closed form transglutaminase shall not be affected.

The open form transglutaminase or transglutaminase stabilized in its open form is used for the detection of open form specific autoantibodies in samples from body fluids, excretions or tissues. The open form transglutaminase or the antigenically active open form TG fragment is used for a more sensitive and more accurate detection of autoimmune diseases and autoimmune disorders.

Preferably the autoimmune disorder is a food protein sensitivity disorder. Further preferably the autoimmune disorder is a cereal protein sensitivity disorder. Further preferably the cereal protein disorder is a gluten sensitivity disorder with or without enteropathy. Most preferably the autoimmune disorder is selected from the group consisting of or comprising celiac disease, dermatitis herpetiformis, Morbus Duhring and gluten sensitive ataxia.

Further preferably the autoimmune disorder is a paraneoplastic neurological syndrome.

Preferably the gluten sensitivity disorder is cerebellar ataxia, peripheral neuropathy, myopathy, ataxia with myoclonus, myelopathy, cerebral calcifications, headache with white matter abnormalities, dementia, chorea or Stiff-person syndrome.

Preferably the autoimmune disorder characterised by neurological dysfunction is anxiety, depression, brainstem encephalitis, cerebral vasculitis, chorea, dementia, epilepsy, cerebral calcifications, headache with white matter abnormalities, neuromyotonia, myasthenia gravis, myopathy, peripheral neuropathy, a paraneoplastic syndrome, progressive multifocal leukoencephalopathy, progressive myoclonic encephalopathy, schizophreniform disorder or stiff-person syndrome.

Most preferably the autoimmune disorder characterised by neurological dysfunction is immune mediated ataxia, encephalitis, cerebral vasculitis, neuromyotonia, myasthenia gravis, polymyositis, immune mediated peripheral neuropathy, a paraneoplastic syndrome, or stiff-person syndrome.

Preferably the autoimmune disorder characterised by neurological dysfunction is ataxia, ataxia with myoclonus, encephalitis, polymyositis, epilepsy with occipital cerebral calcifications, peripheral polyneuropathy, loss of sensation or muscle weakness.

Preferably the neurological dysfunction is characterized by ataxia, ataxia with myoclonus, anxiety, depression, dementia, epilepsy with occipital cerebral calcifications, headache with white matter abnormalities, peripheral polyneuropathy, loss of sensation or muscle weakness.

Preferably the paraneoplastic neurological syndrome is paraneoplastic cerebellar degeneration, paraneoplastic encephalomyelitis, paraneoplastic opsoclonus-myoclonus, cancer associated retinopathy, paraneoplastic stiff-person syndrome, paraneoplastic necrotizing myelopathy, a motor neuron syndrome including amyotrophic lateral sclerosis (ALS) and subacute motor neuronopathy, subacute sensory neuronopathy, autonomic neuropathy, acute sensorimotor neuropathy, polyradiculoneuropathy (Guillain-Barr6), brachial neuritis, chronic sensorimotor neuropathy, a sensorimotor neuropathy associated with plasma cell dyscrasias, vasculitic neuropathy or neuromyotonia.

Preferably the open form TG2 is used for the detection auf autoantibodies in the sera of patients suffering from autoimmune diseases characterized by the presence of autoantibodies against TG2. Moreover the open form TG2 is preferably used for the detection of autoantibodies in samples from patients suffering from a gluten sensitive disorder, especially suffering from celiac disease. A preferred embodiment of the present invention is directed to the use of open form TG2 for the detection of specific autoantibodies in celiac disease patients who have no or low antibody titers against closed form TG2.

In another embodiment of the present invention the open form TG2 is used for the detection of autoantibodies in patients suffering from diabetes mellitus type 1 and in a still further embodiment of the present invention the open form TG2 is used for the detection of specific autoantibodies in recent onset celiac disease patients which did not develop antibodies against closed form TG2 yet.

Preferably the open form TG3 is used for the detection auf autoantibodies in the sera of patients suffering from autoimmune diseases characterized by the presence of autoantibodies against TG3. Moreover the open form TG3 is preferably used for the detection of autoantibodies in samples from patients suffering from dermatitis herpetiformis.

Preferably the open form TG6 is used for the detection auf autoantibodies in the sera of patients suffering from autoimmune diseases characterized by the presence of autoantibodies against TG6. Moreover the open form TG6 is preferably used for the detection of autoantibodies in samples from patients suffering from neuropathy, ataxia or glutensensitive ataxia or stiff person syndrom.

In the present invention any method and device suitable for the diagnostic detection of proteins or antibodies in samples of body fluids or in tissue samples can be used to detect autoantibodies reacting with transglutaminases. Examples of such methods and devices include: EIA/ELISA, LiA, FiA, RIA, IRMA, IEMA/EIA, ILMA, IFMA, immunodiffusion, Western-blot, Dot-blot, immunohistochemistry, protein chips or protein arrays. Thus another aspect of the present invention is directed to protein chips and protein arrays containing at least one transglutaminase or at least one antigenically active open form TG fragment preferably in an immobilized form. Such chips and arrays may preferably contain more than one transglutaminase isoform selected from transglutaminase 1, transglutaminase 2, transglutaminase 3, transglutaminase 4, transglutaminase 5, transglutaminase 6, transglutaminase 7, or coagulation factor XIII, preferably in an immobilized form.

Another aspect of the present invention relates to pharmaceutical formulations and pharmaceutical compositions containing the open form of a TG together with at least one pharmaceutically acceptable carrier, excipient, solvent and/or diluents.

The preferred preparations and formulations are in administrable form which is suitable for oral application or injection. These administrable forms include pills, tablets, film tablets, coated tablets, capsules, solutions, dispersions and deposits.

For example, for oral administration in the form of tablets or capsules, the open form TG may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and colouring agents may also be incorporated in the mixture.

Suitable binders include starch, gelatine, natural sugars, corn sweeteners, natural and synthetic gums such as acacia, sodium alginate, carboxymethyl-cellulose, polyethylene glycol and waxes. Among the lubricants that may be mentioned for use in these dosage forms are boric acid, sodium benzoate, sodium acetate, sodium chloride, and the like. Disintegrants include starch, methylcellulose, guar gum and the like. Sweetening and flavouring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide a controlled release of the open form TG. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions especially for injection. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions especially for injection.

The term capsule refers to a special container or enclosure made of methyl cellulose, polyvinyl alcohols, or denatured gelatines or starch for holding or containing compositions comprising the active ingredients. Hard shell capsules are typically made of blends of relatively high gel strength bone and pork skin gelatines. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refers to the active ingredients dispersed or solubilized in a hydrophilic semi-solid matrix.

These pharmaceutical preparations can be used for a hyposensitisation therapy for patients suffering from autoimmune diseases caused by a gluten intolerance. Consequently, another aspect of the present invention is directed to the use of an open form of a TG, such as TG2, for the preparation of a pharmaceutical composition for hyposensitisation of a patient suffering from an autoimmune disorder caused by a gluten intolerance.

As used herein, the term "hyposensitisation therapy" refers to an immunologic desensitization or allergen-specific immunotherapy, wherein a patient is vaccinated with initially low doses of an open form TG such as TG1, TG2, TG3, TG4, TG5, TG6, TG7 or coagulation factor XIII, which are increased during the treatment period with the aim of inducing immunologic tolerance.

Accordingly the present invention discloses a method for hyposensitizing a mammal including a human by administering a pharmaceutically effective amount of an open form TG to said mammal which suffered from an autoimmune disorder caused by gluten intolerance. The open form TG is preferably administered starting from low doses with increasing doses during the treatment period after a gluten free diet of the patient and at a time where the patient does not show any symptoms of the autoimmune disease which could be measured clinically or by biopsy or serology.

### Description of the Figures

- Figure 1: shows the structure of TG2 - Z-PIMen(OEt)-QPL-OMe-conjugate revealing the open conformation of TG2. N: N-terminal fibroncetin binding domain; Core: catalytic core domain; β1 and β2: beta sheet domains; I: Inhibitor (Z-PIMen(OEt)-QPL-OMe)

### EXAMPLES

### Example 1

### Production of stabilized open form TG2

*Escherichia coli* cells producing recombinant TG2 were centrifuged at 3,700 rpm for 20 minutes. The pellet was resuspended in buffer and lysed by high pressure homogenization. After centrifugation, the supernatant was applied to a column containing Ni-NTA resin (Qiagen, Hilden, Germany). The column was rinsed with buffer until baseline was reached. TG2 was then eluted in a buffer containing 300 mM imidazole. Fractions containing TG2 were pooled and further purified by anion exchange and gel permeation chromatography. Preparation of stabilized open form TG2 for crystallization was performed by incubating freshly prepared TG2 with Z-PIMen(OEt)-QPL-OMe (inhibitor) at a ratio of 1 to 50 at room temperature for 30 min and then at 4°C overnight. Excess inhibitor was removed by anion exchange chromatography. TG2-inhibitor conjugate was concentrated to 8 mg/mL. Glycerol was added to a final concentration of 10%. Storage was performed at -80°C.

### Example 2

### Crystallization and verification of the open form

Crystallization has been performed applying the sitting-drop vapor diffusion method and 1.75 - 2.25 M (NH₄)₂SO₄, 100 mM HEPES pH 6.75 - 7.5 at 18°C.
Rhombic-shaped crystals appeared in 5 to 7 days (Space group: P4₁2₁2; Unit cell constants: a, b = 1.0 Å, c = 310.4 A). The data set was collected with synchrotron radiation and yielded a resolution of 2.5 Å. Processing of the data allowed to determine the position of the inhibitor in the TG2 and confirmed the open conformation.

### Example 3

### Coating of microtiter-plates with open form TG2

Open form TG2 has been produced as described in example 1. Nunc Lockwell Maxisorp plates have been coated overnight at 4°C with 100 µl per well coating solution, composed of 20 mM Tris-HCL, pH 7.4, 150 mM NaCl and 1 µg/ml open form TG2. Then coating solution was removed and plates were washed intensively with TBS (20 mMTris-HCl, pH7.4, 150 mM NaCl) containing 0.01% Tween 20. Blocking was performed by the addition of 200 µl TBS containing 3% bovine serum albumin for 1 h. Blocking solution was removed and plates were washed intensively with TBS (20 mMTris-HCl, pH7.4, 150 mM NaCl) containing 0.01 % Tween 20.

### Example 4

### Analysis of sera

Sera from celiac disease patients and from blood donors have been analysed for antibodies against closed form TG2 and open form TG2 by Enzyme Linked Immuno Sorbent Assays (ELISA). For the detection of IgA-type autoantibodies against closed form TG2 ELISA-Kit E001 from Zedira GmbH, Darmstadt, Germany was used, whereas for IgG-type autoantibodies kit E002 from Zedira GmbH, Darmstadt, Germany was used.

For detection of IgA and IgG-type antibodies against open conformation TG2 the microtiter-plates as described in example 3 have been used. All other components necessary for performing the ELISAs have been taken from Zedira kits E001 and E002 respectively. Accordingly the protocols of E001 and E002 have been used: 10 µl of each serum have been diluted with 990 µl sample buffer, mixed thoroughly and centrifuged to sediment insoluble particles. Immediately prior to use, the solid phase was washed once with 350 µL wash buffer per well. After 10 seconds the wash butter was completely removed. 100 µL of the calibrators, the negative and positive control and the diluted samples have rapidly been dispensed into the micro plate wells. After incubation for 30 minutes at room temperature the wells have been washed 4 times with 350 µl wash buffer. Using an 8-channel pipette 100 µl of conjugate have been dispensed per well subsequently and incubated for 30 min. After a further washing procedure 100 µl substrate solution have been dispensed into the wells and incubated for 30 minutes. Then 100 µl stop solution have been added. Finally the plates were agitated on an orbital shaker for about 10 seconds and the absorbance at 450 nm has been determined.

Results are given in Tab. 1 - 4. Surprisingly the standards (mixture of celiac disease patients sera) yielded significantly higher extinction values at 450 nm when the open form TG2 was used as antigen in the IgG-ELISAs (Tab. 1 and 2). This demonstrates a higher titer of antibodies to open form TG2 in the standards than to closed form TG2. Blood donor's serum 26, which is tested positive in the closed form ELISA is clearly negative in the open form ELISA. Therefore the open form TG2-ELISA provides less false positive results in autoimmune diagnostics (Tab. 2).

**Table 1: anti-IgG-ELISAs using standard TG2 (closed form) and the novel open form TG2 on blood donors sera. Positive values are written in bold letters.**

| anti-IgG ELISAs | Closed form TG2 | | Open form TG2 | |
|---|---|---|---|---|
| blood donors | ΔE450 nm [mOD] | U/mL (Cutoff: 3 U/ml) | ΔE450 nm [mOD] | U/mL (Cutoff: 3 U/ml) |
| Standard 1 | 43 | 0 | 47 | 0 |
| Standard 2 | 162 | 1 | 327 | 1 |
| Standard 3 | 376 | 3 | 733 | 3 |
| Standard 4 | 817 | 10 | 1492 | 10 |
| Standard 5 | 1429 | 30 | 2283 | 30 |
| Standard 6 | 2038 | 100 | 2716 | 100 |
| Serum No | | | | |
| 1 | 137 | 0,79 | 237 | 0,66 |
| 2 | 131 | 0,74 | 174 | 0,44 |
| 3 | 132 | 0,74 | 192 | 0,50 |
| 4 | 148 | 0,88 | 217 | 0,59 |
| 5 | 150 | 0,90 | 239 | 0,67 |
| 6 | 137 | 0,79 | 189 | 0,49 |
| 7 | 112 | 0,58 | 188 | 0,49 |
| 8 | 155 | 0,94 | 222 | 0,61 |
| 9 | 125 | 0,69 | 200 | 0,53 |
| 10 | 97 | 0,45 | 147 | 0,35 |
| 11 | 168 | 1,05 | 190 | 0,50 |
| 12 | 184 | 1,19 | 264 | 0,76 |
| 13 | 154 | 0,93 | 160 | 0,39 |
| 14 | 162 | 1,00 | 219 | 0,60 |
| 15 | 161 | 0,99 | 259 | 0,74 |
| 16 | 252 | 1,80 | 300 | 0,90 |
| 17 | 142 | 0,83 | 201 | 0,53 |
| 18 | 166 | 1,03 | 241 | 0,68 |
| 19 | 130 | 0,73 | 202 | 0,54 |
| 20 | 123 | 0,67 | 164 | 0,40 |
| 21 | 136 | 0,78 | 234 | 0,65 |
| 22 | 171 | 1,08 | 251 | 0,71 |
| 23 | 163 | 1,01 | 238 | 0,67 |
| 24 | 115 | 0,60 | 191 | 0,50 |
| 25 | 143 | 0,84 | 226 | 0,62 |
| 26 | 535 | **4,89** | 380 | 1,21 |
| 27 | 125 | 0,69 | 188 | 0,49 |
| 28 | 161 | 0,99 | 235 | 0,66 |
| 29 | 214 | 1,45 | 296 | 0,88 |
| 30 | 198 | 1,31 | 276 | 0,81 |
| 31 | 183 | 1,18 | 227 | 0,63 |
| 32 | 144 | 0,85 | 214 | 0,58 |
| 33 | 96 | 0,44 | 146 | 0,34 |
| 34 | 151 | 0,91 | 228 | 0,63 |
| 35 | 151 | 0,91 | 241 | 0,68 |
| 36 | 130 | 0,73 | 183 | 0,47 |
| 37 | 140 | 0,81 | 214 | 0,58 |
| 38 | 117 | 0,62 | 170 | 0,43 |
| 39 | 167 | 1,04 | 223 | 0,61 |
| 40 | 168 | 1,05 | 252 | 0,72 |

**Table 2: anti-IgG-ELISAs using standard TG2 (closed form) and the novel open form TG2 on celiac disease patients sera. Positive values are written in bold letters.**

| anti-IgG ELISAs | Closed form TG2 | | Open form TG2 | |
|---|---|---|---|---|
| Patients sera | ΔE450 nm [mOD] | U/mL (Cutoff: 3 U/ml) | ΔE450 nm [mOD] | U/mL (Cutoff: 3 U/ml) |
| Standard 1 | 44 | 0 | 51 | 0 |
| Standard 2 | 161 | 1 | 351 | 1 |
| Standard 3 | 363 | 3 | 763 | 3 |
| Standard 4 | 833 | 10 | 1530 | 10 |
| Standard 5 | 1440 | 30 | 2327 | 30 |
| Standard 6 | 1969 | 100 | 2755 | 100 |
| Serum No | | | | |
| 41 | 201 | 1,36 | 519 | 1,69 |
| 42 | 109 | 0,55 | 159 | 0,35 |
| 43 | 154 | 0,94 | 204 | 0,49 |
| 44 | 176 | 1,14 | 233 | 0,59 |
| 45 | 142 | 0,84 | 461 | 1,44 |
| 46 | 119 | 0,63 | 281 | 0,75 |
| 47 | 112 | 0,57 | 186 | 0,43 |
| 48 | 148 | 0,89 | 284 | 0,76 |
| 49 | 110 | 0,56 | 165 | 0,36 |
| 50 | 700 | **7,57** | 1427 | **8,68** |
| 51 | 976 | **13,09** | 1716 | **12,74** |
| 52 | 130 | 0,73 | 231 | 0,58 |
| 53 | 128 | 0,72 | 462 | 1,44 |
| 54 | 143 | 0,84 | 200 | 0,48 |
| 55 | 90 | 0,39 | 135 | 0,27 |
| 56 | 116 | 0,61 | 164 | 0,36 |
| 57 | 107 | 0,53 | 117 | 0,21 |
| 58 | 88 | 0,37 | 105 | 0,17 |
| 59 | 79 | 0,30 | 92 | 0,13 |
| 60 | 97 | 0,45 | 172 | 0,39 |
| 61 | 100 | 0,47 | 144 | 0,30 |
| 62 | 104 | 0,51 | 139 | 0,28 |
| 63 | 99 | 0,47 | 134 | 0,26 |
| 64 | 102 | 0,49 | 121 | 0,22 |
| 65 | 115 | 0,60 | 129 | 0,25 |
| 66 | 144 | 0,85 | 228 | 0,57 |
| 67 | 559 | **5,43** | 1355 | **7,84** |
| 68 | 365 | 2,98 | 673 | 2,47 |
| 69 | 263 | 1,96 | 795 | **3,20** |
| 70 | 152 | 0,92 | 246 | 0,63 |
| 71 | 90 | 0,39 | 166 | 0,37 |
| 72 | 300 | 2,33 | 325 | 0,91 |
| 73 | 235 | 1,68 | 455 | 1,41 |
| 74 | 89 | 0,38 | 172 | 0,39 |
| 75 | 133 | 0,75 | 385 | 1,13 |
| 76 | 77 | 0,28 | 161 | 0,35 |
| 77 | 115 | 0,60 | 171 | 0,38 |
| 78 | 219 | 1,54 | 302 | 0,83 |
| 79 | 331 | 2,66 | 1093 | **5,34** |
| 80 | 107 | 0,54 | 133 | 0,26 |

Detection of autoantibodies of the IgG-type in patient's sera (Tab. 2) revealed lower mUnit-values in the majority of all sera which were tested negative, enlarging the distance to the cutoff. Sera 50, 51 and 67 have been positive in the closed and open form TG2, but the titers for the open form again have been proved higher for two sera. Whereas Serum 68 was positive in the closed form and negative in the open form ELISA, sera 69 and 79 could be proved positive with the open form ELISA.

In Summary use of open form TG2 as antigen in celiac disease IgG-diagnostics results in a higher diagnostic specificity and sensitivity than closed form TG2.

The results of the IgA-type autoantibody-detection are presented in Tab. 3 (blood donor's sera). Whereas the closed form TG2-ELISA could detect 1 positive serum (26), open form TG2 detected 5 positive sera (4, 17, 25, 26, 27).

From 3 negative patients sera using closed form TG2, 1 could be detected positive using the open form TG2, thus increasing the diagnostic sensitivity (Tab. 4).

With two exceptions higher Unit-values have been measured in patient's sera using the open form TG2. Several patients' sera like 49, 71, 76 or 80 showed more than 100% higher titers in the open form assay. This shows that patients have autoantibodies specific to the open conformation of TG2. Especially when the titers are close to the cut off the open form TG2 yields more reliable diagnostic results (e. g. sera 61 and 70).

The analysis-results of further patients sera are described in the following:
The patient number A is a child, diagnosed for celiac disease by histology upon biopsy. While negative or doubtful results are obtained using standard diagnostics (2.0 U/ml for IgG and 1.2 U/ml for IgA, cut off = 3 U/ml), the novel antigen is able to detect early antibodies. (13 U/ml for IgG and 45 U/ml for IgA)
Patient number B and C presented to the doctor with severe symptoms of celiac disease confirmed by biopsy. However, only comparable low or doubtful titers against TG2 (2.9 or 3.5 U/ml, cutoff = 3 U/ml) have been found. Indeed, the new antigen shows tremendously higher titers correlating with severity of the inflammation and villous atrophy (Patient B, Marsh II by biopsy: 19 U/ml, Patient C, Marsh III by biopsy: 39 U/ml).
Patient D was diagnosed with diabetes type one. While the standard ELISA displays no antibodies against TG2 the novel antigen could detect IgG (11 U/ml) as well as IgA (46 U/ml) antibodies.
Patient E was diagnosed for psoriasis. Whereas no TG2-autoantibodies could be detected with the standard ELISA, clearly positive values (IgA: 37 U/ml, IgG: 21 U/ml) have been obtained using the open form TG2 as antigen in the ELISA.

In conclusion we have shown the presence of autoantibodies of the IgA and the IgG-type which specifically recognize the open conformation TG2. Usage of open form TG2 increases diagnostic sensitivity and specificity in the corresponding autoimmune diagnostics, e.g. celiac disease diagnostics.

Therefore with the newly developed open form TG2 we provide a novel diagnostic antigen which helps to improve TG2-based diagnostics. It may be used in all methods used for the detection of antibodies in samples of body fluids or tissue samples. Examples for such methods include: EIA/ELISA, LiA, FiA, RIA, IRMA, IEMA/EIA, ILMA, IFMA, immunodiffusion, Western-blot or Dot-blot. Any of these methods could be adapted for diagnostic purposes using the antigen described herein in detail by a person skilled in the art.

As transglutaminases are structurally related, the phenomenon surprisingly found for TG2 and described in detail in this application, will also be applicable to other transglutaminases like TG3 in the diagnosis of dermatitis herpetiformis or TG6 in the diagnosis of glutensensitive neurological disorders.

**Table 3: anti-IgA-ELISAs using standard TG2 (closed form) and the novel open form TG2 on healthy donors sera. Positive values are written in bold letters.**

| anti-IgA ELISAs | Closed form TG2 | | Open form TG2 | |
|---|---|---|---|---|
| Blood donors sera | ΔE450 nm [mOD] | U/mL (Cutoff: 3 U/ml) | ΔE450 nm [mOD] | U/mL (Cutoff: 3 U/ml) |
| Standard 1 | 45 | 0 | 45 | 0 |
| Standard 2 | 101 | 1 | 108 | 1 |
| Standard 3 | 209 | 3 | 220 | 3 |
| Standard 4 | 537 | 10 | 568 | 10 |
| Standard 5 | 1161 | 30 | 1264 | 30 |
| Standard 6 | 2185 | 100 | 2322 | 100 |
| Serum No | | | | |
| 1 | 86 | 0,73 | 120 | 1,20 |
| 2 | 84 | 0,70 | 106 | 0,97 |
| 3 | 137 | 1,65 | 177 | 2,20 |
| 4 | 148 | 1,86 | 251 | **3,59** |
| 5 | 80 | 0,62 | 110 | 1,03 |
| 6 | 97 | 0,93 | 172 | 2,11 |
| 7 | 104 | 1,05 | 173 | 2,13 |
| 8 | 115 | 1,25 | 198 | 2,59 |
| 9 | 121 | 1,36 | 166 | 2,00 |
| 10 | 57 | 0,21 | 68 | 0,36 |
| 11 | 78 | 0,59 | 106 | 0,97 |
| 12 | 74 | 0,52 | 117 | 1,15 |
| 13 | 118 | 1,31 | 188 | 2,40 |
| 14 | 89 | 0,78 | 115 | 1,12 |
| 15 | 115 | 1,25 | 145 | 1,63 |
| 16 | 81 | 0,64 | 114 | 1,10 |
| 17 | 128 | 1,49 | 252 | 3,60 |
| 18 | 84 | 0,70 | 107 | 0,98 |
| 19 | 90 | 0,80 | 136 | 1,47 |
| 20 | 70 | 0,45 | 87 | 0,66 |
| 21 | 88 | 0,77 | 168 | 2,04 |
| 22 | 85 | 0,71 | 140 | 1,54 |
| 23 | 87 | 0,75 | 105 | 0,95 |
| 24 | 57 | 0,21 | 72 | 0,42 |
| 25 | 147 | 1,84 | 513 | **8,81** |
| 26 | 212 | **3,06** | 559 | **9,80** |
| 27 | 118 | 1,31 | 226 | 3,11 |
| 28 | 91 | 0,82 | 155 | 1,81 |
| 29 | 121 | 1,36 | 178 | 2,22 |
| 30 | 103 | 1,04 | 160 | 1,89 |
| 31 | 97 | 0,93 | 133 | 1,42 |
| 32 | 71 | 0,46 | 98 | 0,84 |
| 33 | 89 | 0,78 | 113 | 1,08 |
| 34 | 76 | 0,55 | 95 | 0,79 |
| 35 | 100 | 0,98 | 154 | 1,79 |
| 36 | 71 | 0,46 | 76 | 0,49 |
| 37 | 84 | 0,70 | 127 | 1,32 |
| 38 | 62 | 0,30 | 85 | 0,63 |
| 39 | 74 | 0,52 | 117 | 1,15 |
| 40 | 81 | 0,64 | 122 | 1,23 |

**Table 4: anti-IgA-ELISAs using standard TG2 (closed form) and the novel open form TG2 on celiac disease patients sera. Positive values are written in bold letters.**

| anti-IgA ELISAs | Closed form TG2 | | Open form TG2 | |
|---|---|---|---|---|
| Celiac disease patients sera | ΔE450 nm | U/mL | ΔE450 nm | U/mL |
| | [mOD] | (Cutoff: 3 U/ml) | [mOD] | (Cutoff: 3 U/ml) |
| Standard 1 | 45 | 0 | 45 | 0 |
| Standard 2 | 101 | 1 | 108 | 1 |
| Standard 3 | 209 | 3 | 220 | 3 |
| Standard 4 | 537 | 10 | 568 | 10 |
| Standard 5 | 1161 | 30 | 1264 | 30 |
| Standard 6 | 2185 | 100 | 2322 | 100 |
| Serum No | | | | |
| 41 | 262 | **4,00** | 363 | **5,78** |
| 42 | 299 | **4,75** | 479 | **8,09** |
| 43 | 350 | **5,80** | 457 | **7,64** |
| 44 | 579 | **11,22** | 829 | **16,31** |
| 45 | 509 | **9,44** | 690 | **12,73** |
| 46 | 724 | **15,16** | 1160 | **27,02** |
| 47 | 821 | **18,07** | 1041 | **22,76** |
| 48 | 1889 | **72,59** | 2326 | **105,61** |
| 49 | 1174 | **30,91** | 2132 | **86,06** |
| 50 | 1464 | **44,42** | 2050 | **78,51** |
| 51 | 1617 | **53,08** | 2169 | **89,71** |
| 52 | 1865 | **70,57** | 2346 | **107,65** |
| 53 | 1911 | **74,43** | 2487 | **122,04** |
| 54 | 1297 | **36,27** | 1804 | **59,31** |
| 55 | 351 | **5,82** | 431 | **7,12** |
| 56 | 474 | **8,61** | 693 | **12,80** |
| 57 | 414 | **7,21** | 644 | **11,66** |
| 58 | 346 | **5,72** | 542 | **9,39** |
| 59 | 277 | **4,30** | 437 | **7,24** |
| 60 | 87 | 0,79 | 111 | 1,14 |
| 61 | 308 | **4,92** | 501 | **8,53** |
| 62 | 326 | **5,31** | 532 | **9,18** |
| 63 | 193 | 2,70 | 306 | **4,70** |
| 64 | 362 | **6,07** | 570 | **10,00** |
| 65 | 373 | **6,30** | 588 | **10,38** |
| 66 | 83 | 0,71 | 128 | 1,45 |
| 67 | 1665 | **56,14** | 2270 | **99,85** |
| 68 | 3176 | **201,73** | 3354 | **210,44** |
| 69 | 1975 | **79,95** | 2340 | **107,04** |
| 70 | 246 | **3,69** | 360 | **5,74** |
| 71 | 505 | **9,36** | 1261 | **31,03** |
| 72 | 1356 | **39,00** | 1506 | **42,09** |
| 73 | 1372 | **39,78** | 1808 | **59,60** |
| 74 | 409 | **7,10** | 632 | **11,37** |
| 75 | 2126 | **94,57** | 2751 | **148,92** |
| 76 | 309 | **4,95** | 678 | **12,46** |
| 77 | 604 | **11,86** | 898 | **18,26** |
| 78 | 2208 | **102,96** | 2595 | **133,01** |
| 79 | 2365 | **118,98** | 2830 | **157,03** |
| 80 | 284 | **4,45** | 610 | **10,88** |

### Example 5

### Hyposensitisation of a patient

Patient F suffered from celiac disease and has been on gluten free diet for 8 years. According to biopsy and serology Patient F was on remission and showed no celiac disease symptoms any more. Patient F has been injected subcutaneously a solution containing open TG2, which has been produced according to example 1. Injections have been repeated weekly over a period of six months; the doses have been increased over this period in order to reach the maintenance dose. The application has then been repeated applying the maintenance dose once a month for 1.5 years. Patient F was kept on a strict gluten free diet for this whole hyposensitisation therapy period. Later-on the patient started to consume minor amounts (1 g per day) of gluten in addition to the gluten free diet. No celiac disease symptoms could be measured clinically or by biopsy or serology.

Example 5 demonstrates that the open form of a TG can be used for a hyposensitisation therapy for patients suffering from autoimmune diseases caused by a gluten intolerance.

## Claims

1. Method for the diagnosis of an autoimmune disorder, wherein the open form transglutaminase or antigenically active fragments of the open form transglutaminase are used for the detection of the presence of autoantibodies specific for the open form transglutaminase or antigenically active fragments of the open form transglutaminase.

2. Method according to claim 1, wherein the open form transglutaminase or the antigenically active fragment of the open form transglutaminase is selected from TG1, TG2, TG3, TG4, TG5, TG6, TG7 or coagulation factor XIII or antigenically active fragments of TG1, TG2, TG3, TG4, TG5, TG6, TG7 or coagulation factor XIII.

3. Method according to claim 1 or 2, wherein the autoimmune disorder is selected from Addisons's disease, alopecia areata, ankylosing spondylitis, antiphosholipid antibody syndrome, autoimmune hemolytic anemia, autoimmune hepatitis, autoimmune inner ear disease, autoimmune lymphoproliferative syndrome, autoimmune thrombocytopenic purpura, immune thrombocytopenic purpura, balo disease, Behcet's disease, bullous pemphigoid, cardiomyopathy, chronic fatigue immune dysfunction syndrome, chronic inflammatory demyelinating polyneuropathy, Cicatricial pemphigoid, cold agglutinin disease, CREST syndrome, Crohn's disease, Degos disease, dermatomyositis, dermatomyositis-juvenile, discoid lupus, essential mixed cryoglobulinemia, fibromyalgia, Goodpasture's syndrome, Graves disease, Guillain-Baré, Hashimoto thyroiditis, idiopathic pulmonary fibrosis, idiopathic thrombocytopenia purpura, IgA nephropathy, insulin-dependent diabetes, juvneile arthritis, lichen planus, lupus, Ménière disease, mixed connective tissue disease, multiple sclerosis, myasthenia gravis, PANDAS, pemphigus vulgaris, pernicious anemia, polyarteritis nodosa, polychondritis, polyglandular syndromes, polymyalgia rheumatica, polymyositis, dermatomyositis, primary agammaglobulinemia, primary biliary cirrhosis, psoriasis, Raynaud phenomenon, Reiter's syndrome, rheumatic fever, rheumatoid arthritis, sarcoidosis, scleroderma, sclerosing cholangitis, Sjögren's syndrome, Takayasu's arteritis, temporal arteritis, giant cell arteritis, ulcerative colitis, uveitis vasuclitis, vitiligo, Wegener's granulomatosis, neurological disorder, paraneoplastic neurological syndrome, cereal protein sensitivity disorder, gluten sensitive disorders, celiac disease, dermatitis herpetiformis, Morbus Duhring, diabetes mellitus type 1, anxiety, depression, brainstem encephalitis, cerebral vasculitis, chorea, dementia, epilepsy, cerebral calcifications, headache with white matter abnormalities, neuromyotonia, myasthenia gravis, myopathy, peripheral neuropathy, progressive multifocal leukoencephalopathy, progressive myoclonic encephalopathy, schizophreniform disorder, stiff-person syndrome, ataxia, ataxia with myoclonus, encephalitis, polymyositis, epilepsy with occipital cerebral calcifications, peripheral neuropathy, loss of sensation or muscle weakness, paraneoplastic cerebellar degeneration, paraneoplastic encephalomyelitis, paraneoplastic opsoclonus-myoclonus, cancer associated retinopathy, paraneoplastic stiff-man syndrome, paraneoplastic necrotizing myelopathy, a motor neuron syndrome, amyotrophic lateral sclerosis, subacute motor neuronopathy, subacute sensory neuronopathy, autonomic neuropathy, acute sensorimotor neuropathy, polyradiculoneuropathy, brachial neuritis, chronic sensorimotor neuropathy, a sensorimotor neuropathy associated with plasma cell dyscrasias, vasculitic neuropathy and neuromyotonia.

4. Method according to any one of claims 1 - 3, wherein the open form transglutaminase or the antigenically active fragment of the open form transglutaminase is obtained by reacting the not open form transglutaminase or the antigenically active fragment of the not open form transglutaminase with an inhibitor.

5. Method according to claim 4, wherein the inhibitor comprises a backbone, a peptide backbone or peptidomimetic backbone and a thiol reactive group able to form a covalent bond to the thiol group of the cysteine of the TG.

6. Method according to claim 5, wherein the inhibitor is a compound according to general formula [TGl1]:
acceptor-substituted double bond-(CO)ₘ-C₂H₄-backbone [TGl1]
wherein m stands for 0 or 1 and
the acceptor-substituted double bond carries at least one electron-drawing residue capable to conjugate with an electro negativity ≥ 2.20 and
the backbone is a peptide or petidomimetic from at least two amino acids or at least a dipeptidomimetic and/or the backbone shows at least one amide bond.

7. Method for purifying blood, wherein the open form transglutaminase or antigenically active fragments of the open form transglutaminase are used for binding and removing autoantibodies specific for the open form transglutaminase or antigenically active fragments of the open form transglutaminase from the blood.

8. Use of a gluten-derived peptide with a thiol reactive residue or a Michael acceptor residue as an inhibitor for the production of the open form transglutaminase useful for the simultaneous detection of anti-gliadin antibodies and anti open form transglutaminase autoantibodies.

9. Use of the open form of a TG for the preparation of a pharmaceutical composition for hyposensitisation of a patient suffering from an autoimmune disorder caused by a gluten intolerance.

10. Pharmaceutical preparation containing an open form TG together with pharmaceutically acceptable carrier, excipient and/or diluent.

11. A kit for diagnosis of an autoimmune disorder comprising the open form transglutaminase or antigenically active fragments of the open form transglutaminase for the detection of the presence of autoantibodies specific for the open form transglutaminase or the antigenically active fragments of the open form transglutaminase.

12. Kit according to claim 11 further comprising a biotinylated open form transglutaminase for immobilizing the biotinylated open form transglutaminase to a surface coated with biotin or a open form transglutaminase reacted with a biotinylated inhibitor for immobilizing the open form transglutaminase biotinylated inhibitor product to a surface coated with biotin.

13. Kit according to claim 11 further comprising a titer plate or well plate coated with immobilized open form transglutaminase.
